# EUROPEAN PATENT APPLICATION

(11) **EP 0 670 306 A1**
(43) Date of publication of application: **06.09.1995**
(21) Application number: 95102933.9
(22) Date of filing: 02.03.1995
(51) Int. Cl.: C07C 403/24, C12P 23/00

(54) **Process for extracting carotenoids**

(30) Priority: 02.03.1994 JP 32388/94
(71) Applicant: NIPPON OIL CO. LTD., Minato-ku Tokyo (JP)
(72) Inventor: Kitaoka, Motomitsu, Kawasaki-shi, Kanagawa (JP); Tsubokura, Akira, Kawasaki-shi, Kanagawa (JP); Kiyota, Takashi, Yokohama-shi, Kanagawa (JP)
(74) Representative: Cohausz & Florack

(57) **Abstract**

A process for extracting carotenoid compounds comprising the step of bringing a material containing the carotenoid compound into contact with a cyclic hydrophilic organic compound to extract the carotenoid compound from the material to the cyclic hydrophilic organic compound.

By this process, a carotenoid compound can be efficiently extracted resulting in an extract containing a high concentration of the carotenoid compound.

## Description

### BACKGROUND OF THE INVENTION

### 1. Field of Invention

The present invention relates to a process for extracting carotenoid compounds from a material which contains carotenoid compounds, and particularly to a process for extracting carotenoid compounds from a natural product such as microbial cells, algal cells, or tissues or organs of plants or animals.

### 2. Related Art

Carotenoid compounds are a group of red to yellow pigments which are widely distributed, in nature, in microbial cells, algal cells, and the tissues or organs of plants or animals. Recently, carotenoid compounds have been widely used, for example, in the field of food additives as coloring agents for foods and drinks, and as feed additives for coloring the meat, the skin, the eggs etc. of fish such as salmon or trout, and of poultry. In addition, carotenoid compounds are known to have an antioxidation action, and therefore promise to be useful as anti-oxidation agents. It has been found that certain carotenoid compounds have anti-cancer effects, and therefore promise to be useful as ingredients in pharmaceutical compositions.

Carotenoid compounds can be classified as those containing oxygen atom in its molecule, and those not containing oxygen atom, and the former are designated xanthophyll compounds. Among the xanthophyll compounds, astaxanthin, canthaxanthin, zeaxanthin etc. are industrially and synthetically produced. However, considering the trend of a preference for natural products it is becoming difficult to use the synthetic products as food additives and feed additives and also recently it is strongly sought to develop a process for production of natural carotenoid compounds in place of the chemically synthesized products.

Various technics for extraction of carotenoid compound from natural materials have been reported. For example, β-carotene not containing oxygen atom is extracted using petroleum ether etc. as a solvent. However, among carotenoid compounds, those including xanthophyll compounds cannot be efficiently extracted with said solvents. Further, since said solvents are immiscible with water, direct extraction of the xanthophyll compounds from a wet material is not possible.

On the other hand, it is known that xanthophyll compounds can be extracted with chlorinated hydrocarbons such as chloroform. However, since chlorinated hydrocarbons are toxicic or involve potential environmental pollution they cannot, in practice, be used industrially as solvents in the production of foods and feeds. In addition, since they are immiscible with water, direct extraction of xanthophyll compounds from a wet material is impossible.

In addition, processes for extraction of carotenoid compounds with an organic solvent such as acetone, ethyl acetate etc. have been reported (see Japanese Unexamined Patent Publication (Kokai) Nos. 5-155,736; 1-202,261; and 58-88353). However, according to the reported processes, since the concentration of pigments in the extract is low, then a large amount of solvent is required and the extraction processes are highly disadvantageous as an industrial process.

### SUMMARY OF THE INVENTION

The present inventors researched processes for extracting carotenoid compounds accumulated in microbial cells, algal cells, and tissues or organs of plants or animals, and as a result, found that carotenoid compounds can be efficiently extracted with a cyclic hydrophilic organic solvent to obtain an extract containing high concentration of the carotenoid compounds, and completed the present invention. In addition, the present invention is advantageous in that since the cyclic hydrophilic organic compounds used in the present invention are miscible with water, direct extraction of carotenoid compounds from a wet material is possible.

Accordingly, the present invention provides a process for extracting carotenoid compounds characterized by extracting carotenoid compounds from a material containing the carotenoid compounds with a cyclic hydrophilic organic compound. In this case, the cyclic hydrophilic organic compound may be used alone or in combination with an another organic solvent.

According to the present invention, the cyclic hydrophilic organic compounds used as solvents for extracting carotenoid compounds are preferably those compounds which have a 5-membered or 6-membered ring structure as the bone struction and contain one or more oxygen atoms and/or one or more nitrogen atoms in each molecule. The oxygen atom or nitrogen atom may be one which forms part of the ring structure or one which does not form part of the ring structure. The ring structure may have a side chain such as an alkyl group. The cyclic hydrophilic organic compounds used in the present invention are preferably pyridine, tetrahydrofurane, dioxane, cyclohexanone, and the like.

According to the present invention, materials from which carotenoid compounds are to be extracted may be any materials which contain carotenoid compounds. Usually, microbial cells, algal cells, as well as tissues and organs of plants and animals are used. Specifically, there are used microbial cells obtainable by heterotrophically culturing a bacterium capable of producing a carotenoid compound such as E-396 (FERM BP-4283) or Corynebacterium SQH 348 (FERM BP-4284); microbial cells obtainable by heterotrophically culturing a yeast capable of producing a carotenoid compound such as Phaffia rhodozyma; algal cells obtainable by autotrophically culturing in the light an algae such as Haematococcus pluvialis or the genus Dunaliella; crustacea such as krill, crayfishes, crabs, lobsters etc; tissues of plants such as carrot; palm oil, and the like.

Among the microbial cells are preferably mentioned those which are obtained by culturing a bacterium, such as E-396 (FERM BP-4283), which has an ability to produce at least one carotenoid compound selected from the group consisting of astaxanthin, adonixanthin, β-carotene, achinenone, canthaxanthin and zeaxanthin in a medium containing components necessary for the growth, such as a carbon source, a nitrogen source and inorganic salts, as well as, if necessary, substances specially required by the cells, such as vitamins, amino acids, nucleotide bases, and separating the cells by centrifugation, filtration or the like to obtain cultured cells; or culturing a microorganism belonging to the genus Corynebacterium such as (Corynebacterium) SQH348 (FERM-BP-4284) in a medium containing components necessary for the growth of cells, such as a carbon source, a nitrogen source and inorganic salts, as well as, if necessary, substances specially required by the cells, such as vitamins, amino acids, nucleotide bases etc., and separating the cells by centrifugation, filtration etc. to obtain the cultured cells.

Although material containing any amount of a carotenoid compound may be used as a material to be subjected to extraction, preferably the material should contain at least 0.0001% by weight, preferably at least 0.001% by weight, and most preferably at least 0.01% by weight of carotenoid compounds. The upper limit of carotenoid content in a material to be subjected to the extraction is not critical.

Carotenoid compounds to be extracted include, for example, asaxanthin, canthaxanthin, zeaxanthin, adonixanthin, echinenone, cryptoxanthin, adonirubin, asteroidenone, rhodoxanthin, 3-hydroxy echinenone, astaxanthin ester, β-carotene, α-carotene, γ-carotene, lycopene and the like, though the compounds are not limited to them. The present invention is especially useful for the extraction of xanthophyll compounds among carotenoid compounds, which are hardly extracted by conventional processes.

Cyclic hydrophilic organic compounds as extraction solvents may be used singly or in a combination of at least two compounds. A mixture of one or more cyclic hydrophilic organic compounds and another solvent can also be used.

Where one or more cyclic hydrophilic organic compounds are used in a combination with another organic solvent, the mixture should contain at least 10% by weight, preferably at least 40% by weight, and most preferably at least 60% by weight of a cyclic hydrophilic organic compound. Solvents used in the mixed solvent can be, for example, alcohols such as methanol, ethanol, propanol etc.; ketones such as acetone, methylethyl ketone, methyl isopropyl ketone etc.; ether such as diethyl ether, isopropyl ether etc.; esters such as ethyl acetate, butyl acetate etc.; aliphatic hydrocarbons such as hexane, heptane, cyclohexane, petroleum ether, etc.; aromatic hydrocarbons such as benzene, toluene, xylene etc.; chlorinated hydrocarbons such as dichloromethane, chloroform etc., and the like. The solvents are not limited to them above.

Usually, the extraction is carried out by bringing a material to be subjected to extraction into contact with a cyclic hydrophilic organic compound or a mixture of a cyclic hydrophilic organic compound and another solvent. More particularly, a material to be subjected to extraction is dispersed in a cyclic hydrophilic organic compound.

In an embodiment, the contact is made by mixing a material to be subjected to extraction with a cyclic hydrophilic organic compound and agitating or stirring the resulting mixture. During this operation, the carotenoid compounds is extracted from said material to the cyclic hydrophilic organic compounds. Next, the cyclic hydrophilic organic compound is separated from the mixture by any conventional procedure, such as centrifugation, filtration or the like, to obtain an extract containing the carotenoid compounds.

In another embodiment, a column is filled with a material to be subjected to extraction, and a cyclic hydrophilic organic compound or a mixture thereof with another solvent is passed through the column, to obtain an extract containing the carotenoid compounds.

A material to be subjected to extraction may be in a dry condition or a wet condition. Usually, the amount of a cyclic hydrophilic organic compound or a mixture thereof with another solvent is 0.2 to 1000 parts by weight, preferably 0.5 to 500 parts by weight, and most preferably 1 to 100 parts by weight, per 1 part by weight of a material to be subjected to extraction.

The extraction ratio of a carotenoid compounds by a cyclic hydrophilic organic compound or by a mixture of a cyclic hydrophilic organic compound and another solvent is usually at least 60%, and preferably at least 80%.

The extraction temperature is usually between -40°C and 100°C, preferably between -20°C and 80°C, and most preferably between 0°C and 35°C, though it being not limited these temperatures.

The extraction time is usually between one second and 100 hours, preferably between 30 seconds and 10 hours.

The extract containing carotenoid compound is treated to recover the carotenoid compound. Namely, the solvent in the extract is removed by a conventional procedure such as evaporation of the solvent, by lyophilization, vacuum evaporation, flash drying and the like to obtain the desired carotenoid compound in a dried form.

### EXAMPLES

The present invention will be explained more definitely, by referring to the Examples.

### Example 1

An astaxanthin and adonixanthin-producing strain of E-396 (FERM BP-4283 deposited with National Institute of Bioscience & Human Technology Agency of Industrial Science and Technology in Japan) was cultured in 3L of a medium comprising 20 g/L yeast extract, 30 g/L sucrose, 1.5 g/L KH₂PO₄, 1.5g Na₂HPO₄, 0.5 g/L MgSO₄·7H₂O, 0.01 g/L Fe₂(SO₄)₃·7H₂O, and 0.01 g/L CaCl₂ and adjusted with Na₂SO₄ to a pH 7.0 for 5 days, and the culture was centrifuged to obtain 90g of wet cells. The wet cells contained 1.0 mg/g concentration of carotenoid compounds.

To 10g of the wet cells was added 10 mL of pyridine, and extraction was carried out by agitating the mixture for an hour, and to the separated cells was again added 10 mL pyridine, and the mixture was again agitated for an hour. The extraction was further repeated twice. The amount of carotenoid compounds extracted in each extraction is shown in Table 1. As a result, 95% of the total amount of carotenoid compounds was extracted in the first two extractions.

### Example 2

The same procedure as described in Example 1 was repeated except that tetrahydrofurane was used in place of pyridine. The amount of carotenoid compounds extracted in each extraction is shown in Table 1. As a result, 97% of the total amount of carotenoid compounds was extracted in the first two extractions.

### Example 3

The same procedure as described in Example 1 was repeated except that dioxane was used in place of pyridine. The amount of carotenoid compounds extracted in each extraction is shown in Table 1. As a result, 93% of the total amount of carotenoid compositions was extracted in the first two extractions.

### Example 4

The same procedure as described in Example 1 was repeated except that cyclohexanone was used in place of pyridine. The amount of carotenoid compounds extracted in each extraction is shown in Table 2. As a result, 88% of the total amount of carotenoid compounds was extracted in the first two extractions.

### Example 5

The same procedure as described in Example 1 was repeated except that a mixture of tetrahydrofurane and dioxane 1:1 was used in place of pyridine. The amount of carotenoid compounds extracted in each extraction is shown in Table 1. As a result, 96% of the total amount of carotenoid compounds was extracted in the first two extractions.

### Example 6

The same procedure as described in Example 1 was repeated except that a mixture of tetrahydrofurane and cyclohexanone 1:1 was used in place of pyridine. The amount of carotenoid compounds extracted in each extraction is shown in Table 1. As a result, 94% of the total amount of carotenoid compounds was extracted in the first two extractions.

### Example 7

The same procedure as described in Example 1 was repeated except that a mixture of tetrahydrofurane, dioxane and cyclohexanone 2:1:1 was used in place of pyridine. The amount of carotenoid compounds extracted in each extraction is shown in Table 1. As a result, 95% of the total amount of carotenoid compounds was extracted in the first two extractions.

### Example 8

The same procedure as described in Example 1 was repeated except that a mixture of pyridine, tetrahydrofurane, dioxane and cyclohexanone 1:1:1:1 was used in place of pyridine. The amount of carotenoid compounds extracted in each extraction is shown in Table 1. As a result, 97% of the total amount of carotenoid compounds was extracted in the first two extractions.

### Comparative Example 1

The same procedure as described in Example 1 was repeated except that acetone was used in place of pyridine. The amount of carotenoid compounds extracted in each extraction is shown in Table 1. As a result, only 52% of the total amount of the carotenoid compounds was extracted in the four extractions.

### Comparative Example 2

The same procedure as described in Example 1 was repeated except that isopropanol was used in place of pyridine. The amount of carotenoid compounds extracted in each extraction in shown in Table 1. As a result, only 48% of the total amount of carotenoid compounds was extracted in the four extractions.

### Comparative Example 3

The same procedure as described in Example 1 was repeated except that hexane was used in place of pyridine. As a result, no carotenoid compounds were extracted.

### Comparative Example 4

The same procedure as described in Example 1 was repeated except that chloroform was used in place of pyridine. As a result, no carotenoid compounds were extracted.

**Table 1**

| Amount of Carotenoid Compounds Extracted | | | | |
|---|---|---|---|---|
| | Amount of carotenoid compounds extrate (mg) | | | |
| | First ext. | Second ext. | Third ext. | Fourth ext. |
| Example 1 | 6.1 | 3.4 | 0.3 | 0.1 |
| Example 2 | 6.5 | 3.2 | 0.3 | 0.0 |
| Example 3 | 5.9 | 3.4 | 0.4 | 0.1 |
| Example 4 | 5.3 | 3.5 | 0.7 | 0.2 |
| Example 5 | 6.3 | 3.3 | 0.3 | 0.0 |
| Example 6 | 6.2 | 3.4 | 0.3 | 0.1 |
| Example 7 | 6.6 | 2.9 | 0.2 | 0.0 |
| Example 8 | 6.7 | 3.0 | 0.3 | 0.0 |
| Comp. Example 1 | 0.8 | 3.6 | 0.9 | 0.2 |
| Comp. Example 2 | 2.8 | 1.5 | 0.4 | 0.1 |
| Comp. Example 3 | 0.0 | 0.0 | 0.0 | 0.0 |
| Comp. Example 4 | 0.0 | 0.0 | 0.0 | 0.0 |

### Examples 9 to 12

A canthaxanthin producer strain Corynebacterium sp. SQH 348 (FERM BP-4284) was cultured in 3L of a medium containing 30 g/L yeast extract, 10 g/L glucose, 1 mL/L soybean oil, 2.5 g/L NH₄NO₃, 1.5 g/L KH₂PO₄, 1.5 g/L Na₂HPO₄, 0.5 g/L MgSO₄·7H₂O, 0.01 g/L Fe₂(SO₄)₃·7H₂O and 0.01 g/L CaCl₂, adjusted to pH 8.0 with CaCO₃, for 7 days, and the culture was centrifuged to obtain 75g of wet cells. The wet cells contained 0.61 mg/g carotenoid compounds. Next, 10g of the wet cells was subjected to extractions as described in Examples 1 to 4 using different extraction solvents. The result is shown in Table 2.

**Table 2**

| Amount of Carotenoid Compounds Extracted | | | | | |
|---|---|---|---|---|---|
| Extraction solvent | | Amount of carotenoid compounds extracted | | | |
| | | First ext. | Second ext. | Third ext. | Fourth ext. |
| Example 9 | Pyridine | 3.8 | 2.1 | 0.2 | 0.0 |
| Example 10 | Tetrahydrofurane | 3.9 | 2.0 | 0.2 | 0.0 |
| Example 11 | Dioxane | 3.5 | 2.2 | 0.3 | 0.1 |
| Example 12 | Cyclohexanone | 3.1 | 2.4 | 0.5 | 0.1 |

### Example 13

An astaxanthin producer yeast Phaffia rhodozyma was cultured in 3L of MYP medium for 10 days. After finishing the culture, the culture medium was centrifuged and the collected cells were lyophilized to obtain 23g of dry cells. The dry cells contained 0.86 mg/g of carotenoid compounds. 2g of the dry cells was mixed with 10 mL of pyridine in a glass homogenizer, and homogenized for 30 minutes. As a result, 1.60 mg (yield 93%) of the carotenoid compounds was extracted.

### Example 14

The same procedure as described in Example 13 was repeated except that tetrahydrofurane was used in place of pyridine. As a result, 1.61 mg (yield 94%) of the carotenoid compounds was extracted.

### Example 15

The same procedure as described in Example 13 was repeated except that dioxane was used in place of pyridine. As a result, 1.55 mg (yield 90%) of the carotenoid compounds was extracted.

### Example 16

The same procedure as described in Example 13 was repeated except that cyclohexanone was used in place of pyridine. As a result, 1.51 mg (yield 88%) of the carotenoid compounds was extracted.

### Example 17

Dried cells of an astaxanthin producer algae Haematococcus pluvialis were ground in a mortar, and 2g of the ground cells (carotenoid contact 5.3 mg/g) were filled in a column, and pyridine was passed through in the column, at a flow rate of 0.5 mL/min., for 40 min. for extraction. As a result, 10.5 mg (yield 99%) of the carotenoid compounds was extracted.

### Example 18

The same procedure as described in Example 17 was repeated except that tetrahydrofurane was used in place of pyridine. As a result, 10.3 mg (yield 97%) of the carotenoid compounds was extracted.

### Example 19

The same procedure as described in Example 17 was repeated except that dioxane was used in place of pyridine. As a result, 10.4 mg (yield 98%) of the carotenoid compounds was extracted.

### Example 20

The same procedure as described above was repeated except that cyclohexanone was used in place of pyridine. As a result, 10.1 mg (yield 95%) of the carotenoid compounds was extracted.

As can be seen from the above, according to the present invention, carotenoid compounds, especially xanthophyll compounds, can be efficiently extracted from a material containing the carotenoid compounds by using a cyclic hydrophilic organic compound as an extraction solvent to obtain an extract containing a high concentration of carotenoids.

## Claims

1. A process for extracting a carotenoid compound comprising the step of:
bringing a material containing the carotenoid compound into contact with a cyclic hydrophilic organic compound so as to prepare a mixture of said material and said cyclic hydrophilic organic compound resulting in the extraction of the carotenoid compound from said material to said cyclic hydrophilic organic compound.

2. A process according to claim 1, further comprising the step of separating the cyclic hydrophilic organic compound containing the carotenoid compound from said mixture to obtain an extract.

3. A process according to claim 1, further comprising the step of removing the cyclic hydrophilic organic compound from the extract to recover the carotenoid compound.

4. A process according to claim 1, wherein the carotenoid compound to be extracted is a xanthophyll compound.

5. A process according to claim 1, wherein the cyclic hydrophilic organic compound is a solvent selected from the group consisting of pyridine, tetrahydrofurane, dioxane and cyclohexanon.

6. A process according to claim 1, wherein the cyclic hydrophilic organic compound is used in the form of a mixture of the cyclic hydrophilic organic compound and another solvent.

7. A process according to claim 6, wherein said another solvent is an organic solvent selected from the group consisting of methanol, ethanol, isopropanol, acetone, methyl ethyl ketone, methyl isopropyl ketone, diethyl ether, isopropyl ether, ethyl acetate, butyl acetate, hexane, heptane, cyclohexane, petroleum ether, benzene, toluene, xylene, dichloromethane and chloroform.

8. A process according to claim 1, wherein the material containing a carotenoid compound is selected from the group consisting of microbial cells, algal cells, plant tissue and animal tissue.

9. A process according to claim 8, wherein the cells are those of an organism belonging to the genus Corynebacterium, Phaffia, Haematococcus or Dunaliella.

10. A process according to claim 8, wherein the microbial cells are those of an organism belonging to the genus to which the astaxanthin and adonixanthin-producing strain E-396 (FERM BP-4283) belongs.

11. A process according to claim 10, wherein the microbial cells are those of the strain E-396 (FERM BP-4283).

12. A microbial strain E-396 (FERM BP-4283) capable of producing astaxanthin and adonixanthin.
